# EUROPÄISCHE PATENTSCHRIFT

(11) **EP 1 497 267 B1**
(45) Veröffentlichungstag und Bekanntmachung des Hinweises auf die Patenterteilung: **15.10.2008**
(21) Anmeldenummer: 03722413.6
(22) Anmeldetag: 08.04.2003
(51) Int. Cl.: C07D 233/32

(54) **VERFAHREN ZUR HERSTELLUNG VON CHIRALEN IMIDAZOLIDIN-2-ONEN**
METHOD FOR THE PRODUCTION OF CHIRAL IMIDAZOLIDIN-2-ONES
PROCEDE DE PRODUCTION D'IMIDAZOLIDIN-2-ONES CHIRALES

(30) Priorität: 11.04.2002 DE 10215845
(43) Veröffentlichungstag der Anmeldung: 19.01.2005
(73) Patentinhaber: BASF SE, 67056 Ludwigshafen (DE)
(72) Erfinder: ERNST, Hansgeorg, 67346 Speyer (DE); KOPPENHÖFER, Jürgen, 67435 Neustadt (DE); KLEIN, Daniela, 68161 Mannheim (DE)
(86) Internationale Anmeldenummer: PCT/EP2003/003615
(87) Internationale Veröffentlichungsnummer: WO 2003/084933

(56) Entgegenhaltungen:
- WO-A-01/04098
- DREWES S E ET AL: "EPHEDRINE-DERIVED IMIDAZOLIDIN-2-ONES. BROAD UTILITY CHIRAL AUXILIARIES IN ASYMMETRI SYNTHESIS" CHEMISCHE BERICHTE, VERLAG CHEMIE GMBH. WEINHEIM, DE, Bd. 126, 1993, Seiten 2663-2673, XP002931874 ISSN: 0009-2940 in der Anmeldung erwähnt

## Beschreibung

Die vorliegende Erfindung betrifft ein verbessertes Verfahren zur Herstellung von chiralen Imidazolidin-2-onen der allgemeinen Formel I 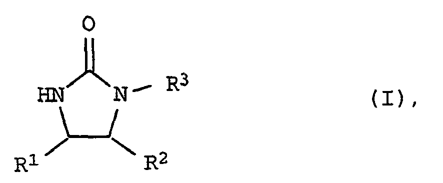 worin
- R¹: für C₁-C₈-Alkyl-, Cyclohexyl-, Phenyl-, einen mit C₁-C₆-Alkyl-, Halo-, Nitro-, C₁-C₆-Alkoxy-, C₁-C₆-Alkylmercapto oder CF₃-substituierten Phenylrest, Naphthyl- oder einen mit C₁-C₆-Alkyl-, Halo-, Nitro-, C₁-C₆-Alkoxy- oder CF₃-substituierten Naphthylrest steht,
- R²: für C₁-C₈-alkyl-, C₁-C₈-Alkenyl-, Cyclohexyl-, Phenyl- oder einen Phenyl-C₁-C₆-alkylrest, der mit einem Nitro-, C₁-C₆-alkoxy-, Methylendioxi- oder CF₃-Rest substituiert sein kann, steht und
- R³: für C₁-C₁₂-Alkyl-, C₁-C₈-Alkenyl-, Cyclohexyl-, Phenyl- oder einen mit C₁-C₆-Alkyl-, Halo-, Nitro-, C₁-C₆-Alkoxy-, Methylendioxi-, Dialkylamin- oder CF₃-substituierten Phenylrest steht,
durch Umsetzung einer Verbindung der allgemeinen Formel II 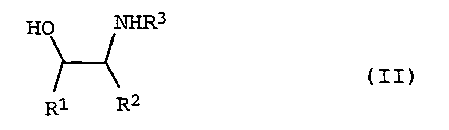 mit Harnstoff in Gegenwart eines Ammoniumsalzes.

Die Herstellung von chiralen Imidazolidin-2-onen, welche wichtige Zwischenprodukte in der asymmetrischen Synthese, insbesondere von biologisch aktiven Verbindungen, darstellen, ist an sich bekannt.

Von besonderer Bedeutung sind dabei Phenyl-substituierte Derivate, die durch Kondensation von Ephedrin mit Harnstoff gewonnen werden.

So beschreibt Close in J. Org. Chem., 15, 1131-1134(1950), die Herstellung von 1,5-Dimethyl-4-phenyl-imidazolidin-2-on durch Kondensation von D,L-Ephedrin Hydrochlorid und Pseudoephedrin mit Harnstoff in der Schmelze. Drewes et al., Chem. Ber., 126, 2663-2673 (1993), beschreiben die Herstellung des entsprechenden (4R,5S)-Enantiomeren ebenfalls durch Kondensation in der Schmelze von L(-)-Ephedrin mit Harnstoff.

Nachteilig ist an dieser Methode jedoch der relativ hohe Anteil an Oxazolidinon als Nebenprodukt, bei entsprechend unbefriedigenden Ausbeuten für das Imidazolidinon.

Aus der WO 01/04098 ist ein Verfahren zur Herstellung von chiralen Imidazolidin-2-onen durch Kondensation von β-Aminoalkoholen mit Harnstoff in Gegenwart eines nichtflüchtigen Ammoniumsalzes bekannt. So kann zum Beispiel L-Ephedrin zu dem entsprechenden 4-Phenyl-Imidazolidin-2-on umgesetzt werden, wie in dem nachfolgenden Reaktionsschema beschrieben.

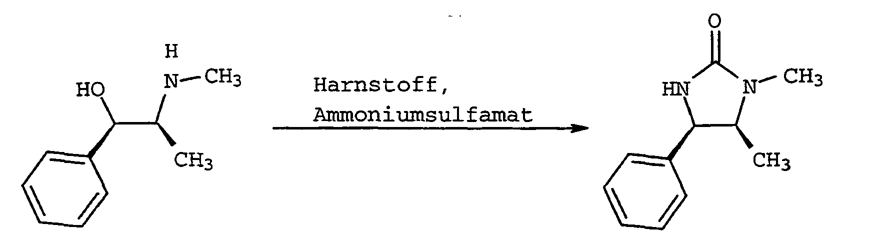

Dabei werden die Einsatzstoffe zunächst unter Einsatz von Toluol als Durchmischungshilfsmittel vermischt, dann das Toluol abdestilliert und die Reaktion in der Schmelze unter Ammoniakentwicklung durchgeführt.

Nachteilig ist dabei unter anderem die starke Ammoniakentwicklung. Das auf diese Weise erhaltene Rohprodukt lässt außerdem hinsichtlich Reinheit und Ausbeute noch zu wünschen übrig.

Aufgabe der vorliegenden Erfindung war es, ein verbessertes Verfahren zu finden, welches auf einfache Weise bei guten Ausbeuten zu Produkten mit hoher Reinheit führt.

Demgemäß wurde ein Verfahren zur Herstellung von chiralen Imidazolidin-2-onen der allgemeinen Formel I 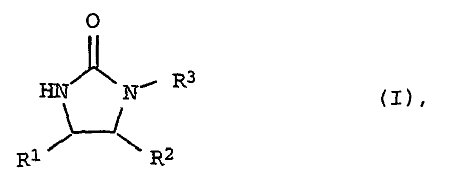 worin
- R¹: für C₁-C₈-Alkyl-, Cyclohexyl-, Phenyl-, einen mit C₁-C₆-Alkyl-, Halo-, Nitro-, C₁-C₆-Alkoxy-, C₁-C₆-Alkylmercapto- oder CF₃-substituierten Phenylrest, Naphthyl- oder einen mit C₁-C₆-Alkyl-, Halo-, Nitro-, C₁-C₆-Alkoxy- oder CF₃-substituierten Naphthylrest steht,
- R²: für C₁-C₈-Alkyl-, C₁-C₈-Alkenyl-, Cyclohexyl-, Phenyl- oder einen Phenyl-C₁-C₆-alkylrest, der mit einem Nitro-, C₁-C₆-Alkoxy-, Methylendioxi- oder CF₃-Rest substituiert sein kann, steht und
- R³: für C₁-C₁₂-Alkyl-, C₁-C₈-Alkenyl-, Cyclohexyl-, Phenyl- oder einen mit C₁-C₆-Alkyl-, Halo-, Nitro-, C₁-C₆-Alkoxy-, Methylendioxi-, Dialkylamin- oder CF₃-substituierten Phenylrest steht,
durch Umsetzung einer Verbindung der allgemeinen Formel II oder deren Salz 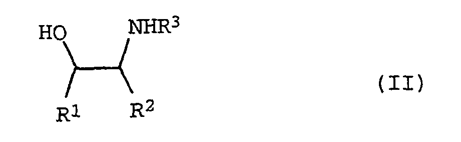 worin R¹, R² und R³ die oben stehende Bedeutung haben,
mit Harnstoff in Gegenwart eines Ammoniumsalzes gefunden, welches dadurch gekennzeichnet ist, dass die Umsetzung in Gegenwart eines polaren organischen Lösungsmittel durchgeführt wird.

Bevorzugt wird ein aprotisches polares Lösungsmittel und besonders bevorzugt N-Methylpyrrolidon eingesetzt.

Gemäß einer weiteren bevorzugten Ausführungsform erfolgt die Reaktion in Gegenwart eines Protonenlieferanten.

Bevorzugt steht R¹ für Phenyl und R², R³ für Methyl, d.h. bevorzugt werden 1R,2S-Ephedrin, 1S,2R-Ephedrin, 1R,2R-Pseudoephedrin oder 1S,2S-Pseudoephedrin oder Salze davon eingesetzt. Als Salze kommen insbesondere die Hydrochloride in Betracht. Ganz besonders bevorzugt sind 1R,2S-Ephedrin, 1S,2R-Ephedrin und die entsprechenden Hydrochloride.

Die Umsetzung erfolgt in Gegenwart von Ammoniumsalzen. Geeignete Ammoniumsalze sind die der Mineralsäuren, der Mineralsäurederivate wie Amidoschwefelsäure oder Amidosulfonsäure oder Ammoniumsalze von gesättigten C₁-C₆-Carbonsäuren.

Als Ammoniumsalze kommen beispielsweise Ammoniumsulfamat, Ammoniumsulfat, Diammoniumhydrogenphosphat, Ammoniumdihydrogenphosphat oder Ammoniumacetat in Betracht, bevorzugt anorganische Ammoniumsalze, wobei Ammoniumsulfamat besonders bevorzugt ist. Das Ammoniumsalz wird in Mengen von 0,5 bis 3 Äquivalenten, bevorzugt 0,9 bis 1,1 Äquivalenten eingesetzt.

Harnstoff wird in Mengen von 1 bis 5 Äquivalenten, bevorzugt 2,5 bis 3,5 Äquivalenten, eingesetzt.

Die Umsetzung erfolgt bevorzugt in Gegenwart einer Protonen liefernden Verbindung. Als Protonenlieferanten kommen starke Säuren, bevorzugt mit einem pKₛ-Wert ≤ 3, in Betracht, beispielsweise Mineralsäuren wie Schwefelsäure, Phosphorsäure, Salzsäure oder Schwefelsäurederivate wie Amidoschwefelsäure sowie organische Säuren wie Sulfonsäuren oder Carbonsäuren beispielsweise Trichloressigsäure oder Trifluormethansulfonsäure. Es können auch Gemische eingesetzt werden.

Als besonders bevorzugte Protonenlieferanten werden p-Toluolsulfonsäure oder Amidoschwefelsäure oder deren Gemische eingesetzt.

Der Protonenlieferant kann in Mengen von 0,05 bis 0,6 Äquivalenten, bevorzugt 0,1 bis 0,5 Äquivalenten, bezogen auf die Verbindung II, eingesetzt werden.

Die Reaktion erfolgt in Gegenwart eines polaren organischen Lösungsmittels, wobei als Lösungsmittel beispielsweise Dimethylsulfoxid, Dimethylformamid, N,N'-Dimethylimidazolidinon oder Ethylenglykol in Betracht kommen, vorzugsweise sind aprotische polare Lösungsmittel. Als besonders bevorzugtes Lösungsmittel wird N-Methylpyrrolidon (NMP) eingesetzt.

Es empfiehlt sich das Lösungsmittel in solchen Mengen einzusetzen, dass das Wertprodukt mit guter Ausbeute und hoher Reinheit direkt aus der Reaktionsmischung auskristallisiert werden kann, ohne dass das Lösungsmittel vorher abdestilliert werden muß, um die Reaktionsmischung aufzukonzentrieren.

Bevorzugt werden pro Mol der Verbindung II 150 bis 250 ml Lösungsmittel zugegeben.

Die festen Ausgangsstoffe können in dem Reaktionsgefäß vorgelegt werden und dann mit dem Lösungsmittel versetzt werden.

Anschließend wird das Gemisch auf Temperaturen im Bereich von 170 bis 190°C, bevorzugt 175 bis 180°C erhitzt. Dabei bildet sich eine klare Lösung.

Im allgemeinen arbeitet man bei Normaldruck. Es kann sich aber auch empfehlen bei erhöhten Drücken zu arbeiten.

Die Reaktionszeit richtet sich nach den eingesetzten Mengen. Das Ende der Reaktion kann durch HPLC-Analytik bestimmt werden.

Anschließend wird die Reaktionsmischung auf Temperaturen im Bereich von 130°C abgekühlt und Wasser zu dem Reaktionsgemisch gegeben. Hierbei bilden sich zwei Phasen. Bei 90 bis 100°C wird dann die Phasentrennung vorgenommen.

Die Menge an zugesetztem Wasser wird so gewählt, dass sich mit dem Reaktionsgemisch zwei Phasen bilden. Die Menge kann je nach verwendetem organischen Lösungsmittel differieren.

Vorzugsweise wird im Falle von NMP soviel Wasser zugesetzt, dass das Verhältnis von NMP zu Wasser 1:1,5 bis 1:3 beträgt, besonders bevorzugt 1:1,8 bis 1:2,3.

Die obere Phase wird weiter gekühlt, wobei im Bereich von 65°C Kristallbildung einsetzt. Man kühlt weiter auf Temperaturen 10±5°C ab, wobei es sich empfiehlt, die kristalline Masse noch nachzurühren.

Anschließend können die Kristalle über ein Filter abgesaugt und mit kaltem Wasser gewaschen werden. Die Trocknung des kristallinen Produkts kann dann im Vakuum erfolgen.

Das so erhaltene Rohprodukt weist im allgemeinen Reinheiten von >90 Gew.-% auf.

Wird eine größere Reinheit gewünscht, so kann das Produkt aus einem geeigneten Lösungsmittelgemisch) rekristallisiert werden. Als Lösungsmittel eignen sich beispielsweise Acetonitril/WasserGemische.

Nach dem modifizierten Herstellungsverfahren wird das Imidazolidinon mit einem deutlich vereinfachten Verfahren mit einer verbesserten Reinheit und Ausbeute hergestellt.

Vorteilhaft ist unter anderen, dass eine Ammoniakentwicklung nahezu vollständig vermieden wird. Ebenso wir auch eine Sublimation des Harnstoffs, wie sie bei der Umsetzung in der Schmelze auftritt, deutlich verringert.

Wegen der guten Reinheit des Rohprodukts ist eine weitere Umkristallisation in der Regel überflüssig.

### Beispiele

### Allgemeine Vorschrift

### Herstellung von 1,5-Dimethyl-4-phenylimidazolidin-2-on

1 Mol eines Ephedrins, 1,025 mol Ammoniumsulfamat, 3 mol Harnstoff und die Protonen liefernde Verbindung wurden in 200 ml NMP vorgelegt. Es wurde auf 175 bis 180°C erhitzt und 2,5 h bei dieser Temperatur gerührt. Danach wurde das Reaktionsgemisch auf 130°C abgekühlt und bei dieser Temperatur Wasser zum Reaktionsgemisch getropft. Es bildeten sich zwei Phasen. Die Unterphase wurde bei 95°C abgetrennt. Die Oberphase wurde weiter auf zunächst 65°C abgekühlt. Bei dieser Temperatur bildeten sich Kristalle. Es wurde weiter auf 10°C abgekühlt und noch 1 h bei dieser Temperatur nachgerührt. Die Kristalle wurden über ein Filter abgesaugt und noch zweimal mit kaltem Wasser gewaschen. Der Feststoff wurde im Vakuum bei RT über Nacht getrocknet.

Die Reinheit des kristallinen Produkts wurde durch HPLC bestimmt.

Weiter Einzelheiten sind der nachstehenden Tabelle zu entnehmen

| Beispiel Nr. | | Protonenlieferant | Wasser [ml] | Ausbeute [%d.Th.] | Gehalt [Gew.-%] |
|---|---|---|---|---|---|
| 1 | (-)-Ephedrin | 10 mol-% p-Tos-OH | 360 | 56 | 98,2 |
| 2 | (+)-Ephedrin | 10 mol-% p-Tos-OH | 560 | 65 | 96,4 |
| 3 | (+)-Ephedrin x HCl | 10 mol-% p-Tos-OH | 360 | 63 | 98,1 |
| 4 | (+)-Ephedrin x HCl | 10 mol-% Amidoschwefels. | 360 | 72 | 98,9 |
| 5 | (-)-Ephedrin | 50 mol-% Amidoschwefels. | 360 | 72 | 98,6 |
| 6 | (+)-Ephedrin x HCl | 10 mol-% p-Tos-OH 40 mol-%Amidoschwefels. | 360 | 76 | 98,5 |
| 7 | (-)-Ephedrin | 10 mol-% Amidoschwefels. | 360 | 70 | 99,6 |
| 8 | (+)-Ephedrin | 10 mol-% Amidoschwefels. | 360 | 67 | 96,5 |
| 9 | (+)-Ephedrin x HCl | -- | 360 | 71 | 99,4 |

### Vergleichsbeispiel 1 (gemäß WO 01/04098)

In einem Rührreaktor wurden 1 eq (-)-Ephedrin, 3 eq Harnstoff und 1,025 eq Ammoniumsulfamat in 0,410 1 Toluol vorgelegt. Es wurde auf Rückfluss erhitzt und das Lösungsmittel abdestilliert. Danach wurde die verbleibende Schmelze 1,5 h auf 175 bis 180°C erhitzt. Man beobachtete eine deutliche Ammoniakausgasung und Harnstoffsublimation. Zur Aufarbeitung des Reaktionsgemisches wurde die Reaktionsmischung auf 105°C heruntergekühlt und 0,31 1 Wasser hinzugegeben. Es wurde weiter auf 57°C abgekühlt und 0,034 1 Ethanol zum Reaktionsgemisch hinzugegeben. Anschließend wurde noch für 4 h bei Raumtemperatur gerührt und der Feststoff dann abfiltriert. Der Feststoff wurde nochmals mit Wasser gewaschen und dann bei RT über 14 h im Vakuum bei 15 mbar getrocknet. Man erhielt ein Rohprodukt mit einer Ausbeute von 69 %. Die Reinheit des Rohproduktes lag bei 84 Gew.-%.

Das Rohprodukt wurde aus einem Wasser/Acetonitrilgemisch umkristallisiert. Danach wurden Reinheiten von > 98 Gew.-% erzielt, wobei die Ausbeute für die Kristallisation bei 75 % lag, was einer Gesamtausbeute von 49 % entspricht.

### Vergleichsbeispiel 2

Analog zu Vergleichsbeispiel 1 wurde (+)-Ephedrin Hydrochlorid umgesetzt. Die Rohausbeute betrug 59 %, bei einer Reinheit von 78 Gew.-%

## Patentansprüche

1. Verfahren zur Herstellung von chiralen Imidazolidin-2-onen der allgemeinen Formel I 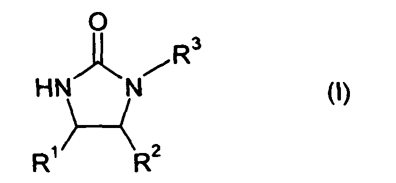 worin
R¹ für C₁-C₈-Alkyl, Cyclohexyl-, Phenyl-, einen mit C₁-C₆-Alkyl-, Halo-, Nitro-, C₁-C₆-Alkoxy-, C₁-C₆-Alkylmercapto- oder CF₃-substituierten Phenylrest, Naphthyl- oder einen mit C₁-C₆-Alkyl-, Halo-, Nitro-, C₁-C₆-Alkoxy- oder CF3-substituierten Naphtkylrest steht,
R² für C₁-C₈-Alkyl-, C₁-C₈-Alkenyl-, Cyclohexyl-, Phenyl- oder einen Phenyl-C₁-C₆-alkylrest, der mit einem Nitro-, C₁-C₆-alkoxy-, Methylendioxi- oder CF3-Rest substituiert sein kann, steht und
R³ für C₁-C₁₂Alkyl-, C₁-C₈-Alkenyl-, Cyclohexyl-, Phenyl- oder einen mit C₁-C₆-Alkyl-, Halo-, Nitro-, C₁-C₆-Alkoxy-, Methylendioxi-, Dialkylamine- oder CF3-substituierten Phenylrest steht,
durch Umsetzung einer Verbindung der allgemeinen Formel 11 oder deren Salz 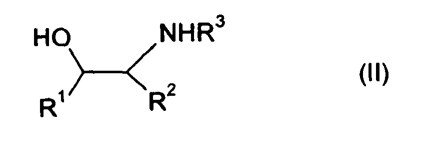 worin R¹, R² und R³ die oben stehende Bedeutung haben,
mit Harnstoff in Gegenwart eines Ammoniumsalzes, **dadurch gekennzeichnet, dass** die Umsetzung in Gegenwart eines polaren organischen Lösungsmittels durchgeführt wird, und dass die Umsetzung in Lösung bei Temperaturen von 170 bis 190°C erfolgt, und dass die Umsetzung in Gegenwart von Protonenlieferanten durchgeführt wird, wobei als Protonenlieferant eine Säure mit einem pKs-Wert ≤ 3 verwendet wird.

2. Verfahren nach Anspruch 1, **dadurch gekennzeichnet, daß** ein aprotisches Lösungsmittel verwendet wird.

3. Verfahren nach einem der Ansprüche 1 oder 2, **dadurch gekennzeichnet, dass** als organisches Lösungsmittel N-Methylpyrro-lidon eingesetzt wird.

4. Verfahren nach einem der Ansprüche 1 bis 3, **dadurch gekennzeichnet, dass** R¹ für Phenyl und R² und R³ für Methyl stehen.

5. Verfahren nach einem der Ansprüche 1 bis 4, **dadurch gekennzeichnet, dass** als Protonenlieferant para-Toluolsulfonsäure eingesetzt wird.

6. Verfahren nach einem der Ansprüche 1 bis 5, **dadurch gekennzeichnet, dass** als Protonenlieferant Amidoschwefelsäuresäure eingesetzt wird.

7. Verfahren nach einem der Ansprüche 1 bis 6, **dadurch gekennzeichnet, dass** der Protonenlieferant in Mengen von 0,05 bis 0,6 Äquivalenten, bezogen auf die Verbindung der allgemeinen Formel II, eingesetzt wird.

8. Verfahren nach einem der Ansprüche 1 bis 7, **dadurch gekennzeichnet, dass** als Verbindung der allgemeinen Formel II (1S,2R)-Ephedrin oder ein Salz davon eingesetzt wird.

9. Verfahren nach einem der Ansprüche 1 bis 8, **dadurch gekennzeichnet, dass** als Verbindung der allgemeinen Formel II (1R,2S)-Ephedrin oder ein Salz davon eingesetzt wird.

## Claims

1. A process for preparing chiral imidazolidin-2-ones of the general formula I 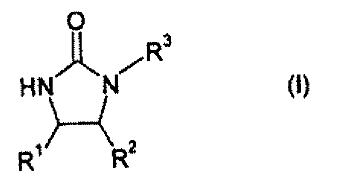 in which
R¹ is C₁-C₈-alkyl, cyclohexyl, phenyl, a C₁-C₆-alkyl-, halo-, nitro-. C₁-C₆-alkoxy-, C₁-C₆-alkylmercapto- or CF₃-substituted phenyl radical, naphthyl or a C₁-C₆-alkyl-, halo-, nitro-, C₁-C₆-alkoxy- or CF₃-substituted naphthyl radical,
R² is C₁-C₈-alkyl, C₂-C₈-alkenyl, cyclohexyl, phenyl or a phenyl-C₁-C₆-alkyl radical which may be substituted by a nitro, C₁-C₆-alkoxy, methylenedioxy or CF₃ radical, and
R³ is C₁-C₁₂-alkyl, C₂-C₈-alkenyl, cyclohexyl, phenyl or a C₁-C₆-alkyl-, halo-, nitro-, C₁-C₆-alkoxy-, methylenedioxy-, dialkylamino- or CF₃-substituted phenyl radical,
by reacting a compound of the general formula II or the salt thereof 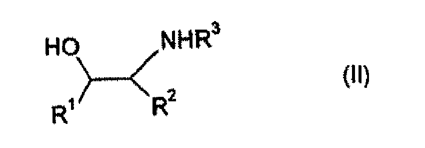 in which R¹, R² and R³ have the abovementioned meaning,
with urea in the presence of an ammonium salt, wherein the reaction is carried out in the presence of a polar organic solvent and the reaction takes place in solution at temperatures of from 170 to 190°C and the reaction is carried out in the presence of proton donors, wherein an acid with a pKa of ≤ 3 is used as proton donor.

2. The process according to claims 1, wherein an aprotic solvent is used.

3. The process according to either of claims 1 or 2, wherein N-methylpyrrolidone is employed as organic solvent.

4. The process according to any of claims 1 to 3, wherein R¹ is phenol and R² and R³ are methyl.

5. The process according to any of claims 1 to 4, wherein para-toluenesulfonic acid is employed as proton donor.

6. The process according to any of claims 1 to 5, wherein sulfamic acid is employed as proton donor.

7. The process according to any of claims 1 to 6, wherein the proton donor is employed in amounts of from 0.05 to 0.6 equivalent based on the compound of the general formula II.

8. The process according to any of claims 1 to 7, wherein (lS,2R)-ephedrine or a salt thereof is employed as compound of the general formula II.

9. The process according to any of claims 1 to 8, wherein (1R,2S)-ephedrine or a salt thereof is employed as compound of the general formula II.

## Revendications

1. Procédé de préparation d'imidazolidin-2-ones chirales de formule générale I 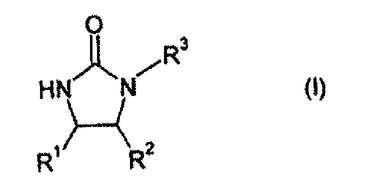 dans laquelle :
R¹ représente un radical alkyle en C₁-C₈, un radical cyclohexyle, un radical phényle, un radical phényle substitué par un groupe alkyle en C₁-C₆, par un groupe halogéno, par un groupe nitro, par un groupe alcoxy en C₁-C₆, par un groupe alkylmercapto en C₁-C₆ ou par un groupe CF₃, un radical naphtyle ou un radical naphtyle substitué par un groupe alkyle en C₁-C₆, par un groupe halogène, par un groupe nitro, par un groupe alcoxy en C₁-C₆ ou par un groupe CF₃,
R² représente un radical alkyle en C₁-C₈, un radical alcényle en C₂-C₈, un radical cyclohexyle, un radical phényle ou un radical phényl-C₁-C₆-alkyle, qui peut être substitué par un radical nitro, un radical alcoxy en C₁-C₆, un radical méthylènedioxy ou un radical CF₃, et
R³ représente un radical alkyle en C₁-C₁₂, un radical alcényle en C₂-C₈, un radical cyclohexyle, un radical phényle ou un radical phényle substitué par un groupe allyle en C₁-C₆, par un groupe halogène, par un groupe nitro, par un groupe alcoxy en C₁-C₆, par un groupe méthylènedioxy, par un groupe dialkylamino ou par un groupe CF₃,
par la réaction d'un composé de formule générale II ou de son sel 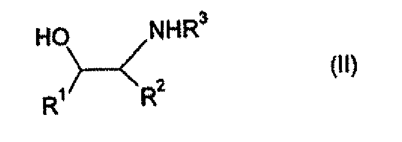 dans laquelle R¹, R² et R³ présentent la signification mentionnée ci-dessus,
avec de l'urée en présence d'un sel d'ammonium, **caractérisé en ce que** la réaction est effectuée en présence d'un solvant organique polaire, que la réaction a lieu en solution à des températures de 170 à 190°C, et que la réaction est effectuée en présence de donneurs de protons, en utilisant un acide présentant une valeur de pKs ≤ 3 en tant que donneur de protons.

2. Procédé selon la revendication 1, **caractérisé en ce que** l'on utilise un solvant aprotique.

3. Procédé selon l'une quelconque des revendications 1 ou 2, **caractérisé en ce que** l'on utilise la N-méthylpyrrolidone en tant que solvant organique.

4. Procédé selon l'une quelconque des revendications 1 à 3, **caractérisé en ce que** R¹ représente un groupe phényle, et R² et R³ représentent un groupe méthyle.

5. Procédé selon l'une quelconque des revendications 1 à 4, **caractérisé en ce que** l'on utilise l'acide para-toluène sulfonique en tant que donneur de protons.

6. Procédé selon l'une quelconque des revendications 1 à 5, **caractérisé en ce que** l'on utilise l'acide amidosulfurique en tant que donneur de protons.

7. Procédé selon l'une quelconque des revendications 1 à 6, **caractérisé en ce que** le donneur de protons est utilisé en des quantités de 0,05 à 0,6 équivalant, par rapport au composé de formule générale II.

8. Procédé selon l'une quelconque des revendications 1 à 7, **caractérisé en ce que** l'on utilise la (1S,2R)-éphédrine, ou un sel de celle-ci, en tant que composé de formule générale II.

9. Procédé selon l'une quelconque des revendications 1 à 8, **caractérisé en ce que** l'on utilise la (1R,2S)-éphédrine, ou un sel de celle-ci, en tant que composé de formule générale II.
